# EUROPEAN PATENT SPECIFICATION

(11) **EP 1 893 775 B1**
(45) Date of publication and mention of the grant of the patent: **07.08.2013**
(21) Application number: 06766347.6
(22) Date of filing: 20.06.2006
(51) Int. Cl.: C07K 14/705, C12Q 1/68

(54) **ALTERNATIVE SPLICING ISOFORM OF LOX-1 PROTEIN ENCODING GENE, AND USES THEREOF**
ALTERNATIVE SPLEISSISOFORM DES FÜR DAS LOX-1-PROTEIN CODIERENDEN GENS UND VERWENDUNGEN DAVON
ISOFORME D'EPISSAGE ALTERNATIVE DE GENE CODANT POUR LA PROTEINE LOX1, ET UTILISATIONS CORRESPONDANTES

(30) Priority: 23.06.2005 IT RM20050325
(43) Date of publication of application: 05.03.2008
(73) Proprietor: Universita' degli Studi di Roma " Tor Vergata", 00173 Roma (IT)
(72) Inventor: NOVELLI, Giuseppe Dip. Medicina di Laboratorio, 00133 Roma (IT); MANGO, Ruggero, 00133 Roma (IT); ROMEO, Francesco Universita degli Studi di Roma "Tor Vergata", 00173 Roma (IT); LAURO, Renato Universita degli Studi di Roma "Tor Vergata", 00173 Roma (IT); CLEMENTI, Fabrizio, 00153 Roma (IT)
(74) Representative: Gitto, Serena
(86) International application number: PCT/IT2006/000470
(87) International publication number: WO 2006/137101

(56) References cited:
- US-A1- 2002 193 567
- TATSUGUCHI MARIKO ET AL: "Oxidized LDL receptor gene (OLR1) is associated with the risk of myocardial infarction." BIOCHEMICAL AND BIOPHYSICAL RESEARCH COMMUNICATIONS. 28 MAR 2003, vol. 303, no. 1, 28 March 2003 (2003-03-28), pages 247-250, XP002414538 ISSN: 0006-291X
- CHEN QI ET AL: "Genetic variation in lectin-like oxidized low-density lipoprotein receptor 1 (LOX1) gene and the risk of coronary artery disease." CIRCULATION, vol. 107, no. 25, 1 July 2003 (2003-07-01), pages 3146-3151, XP002414539 ISSN: 0009-7322
- MANGO R ET AL: "Association of single nucleotide polymorphisms in the oxidised LDL receptor 1 (OLR1) gene in patients with acute myocardial infarction." JOURNAL OF MEDICAL GENETICS. DEC 2003, vol. 40, no. 12, December 2003 (2003-12), pages 933-936, XP002414540 ISSN: 1468-6244 cited in the application
- KATAOKA H ET AL: "Expression of lectinlike oxidized low-density lipoprotein receptor-1 in human atherosclerotic lesions" CIRCULATION, LIPPINCOT WILLIAMS AND WILKINS, BALTIMORE, US, vol. 99, no. 24, 22 June 1999 (1999-06-22), pages 3110-3117, XP002311437 ISSN: 1524-4539 cited in the application
- MANGO RUGGIERO ET AL: "In vivo and in vitro studies support that a new splicing isoform of OLR1 gene is protective against acute myocardial infarction." CIRCULATION RESEARCH. 22 JUL 2005, vol. 97, no. 2, 22 July 2005 (2005-07-22), pages 152-158, XP002414541 ISSN: 1524-4571

## Description

The present invention relates to a new isoform of alternative splicing of the OLR1 gene encoding for the LOX-1 protein, uses and methods related to the treatment and to the prediction of the risk of cardiovascular pathologies. More particularly, the present invention refers to methods for the prediction of the risk to develop cardiovascular pathologies, such as atherosclerosis, myocardial infarction, coronary artery disease, cerebral stroke, ischemia, acute and chronic peripheral vascular pathologies.

The atherosclerosis is a multifactorial disease with genetic component that represents the principal cause of mortality and morbidity in the industrialized countries. The main clinical manifestations of the atherosclerosis are represented by coronary artery disease (CAD), acute myocardial infarction (AMI), cerebral stroke and peripheral vascular disease. Recent studies have shown that atherogenesis is no longer an inevitable consequence of aging but instead is a chronic disease whose genesis both individual factors, mainly with a strong genetic component (hypertension, hypercholesterolemia, diabetes) and environmental (smoking, diet, stress) contribute to (Binder CJ, et al., Nat Med. 8; 1218-1226; Steinberg, D. Nat Med. 8; 1211-1217).

Recently several epidemiological studies highlighted newly defined non-traditional risk factors that are emerging as being equally important in atherosclerosis pathogenesis (Steinberg, D. Nat Med. 8; 1211-1217). Among these oxidized low-density lipoprotein (OxLDL) are considered to play a fundamental role in the entire process of atherogenesis. OxLDL elicits endothelial dysfunction, a key step in the initiation of atherosclerosis plaque, favouring generation of reactive oxygen species (free raduicals), inhibition of nitric oxide synthesis, and enhancement of monocytes adhesion to activated endothelial cells (Mehta JL and Them D. J Am Coll Cardiol. 39; 1429-1435; Chen M, et al., Pharmacol Ther. 95; 89-10).

In addition, OxLDL are involved in inducing smooth muscle cell migration and proliferation, and in foam cells formation, crucial events of atherogenesis (Chen M et al., Pharmacol Ther. 95; 89-10). Increased plasmatic levels of OxLDL were associated to plaque instability and to acute coronary syndromes (MI and unstable angina). In fact as shown, plasmatic OxLDL levels show a significant positive correlation with the severity of acute coronary syndromes and atherosclerotic lesions. Instable plaques contain contain significantly higher percentage of OxLDL-positive macrophages (Ehara S, et al., Circulation. 103; 1955-1960) and of apoptotic cells. According to this scenario, it has been shown that OxLDL are cytotoxic by inducing necrosis and apoptosis of different cells involved in the process of atherogenesis (smooth muscle cells, endothelial cells, and macrophages)(Chen J, et al., Circ Res. 94; 269-270; Hardwick SJ, et al., J Pathol. 179; 294-302; Sata M, et al., J Clin Invest. 102; 1682-1682; Lee TS and Lee YC, Am J Physiol. 280; 709-718). These processes have been proposed to lead to atherosclerotic plaque destabilization and subsequent rupture with acute atherothrombotic vascular occlusion and tissue infarction.

Most of the above mentioned effects are determined by the interaction of OxLDL with their major receptor, named LOX-1. LOX-1 receptor is encoded from the OLR1 gene. Such gene, mapped on the 12p12.3-13.2 chromosomal region, is constituted by 6 exons and 5 introns.

LOX-1 is a type-II membrane protein belonging to the C-type lectin family. LOX-1 consists of 4 domains: a short N-terminal cytoplasmic domain, a transmembrane region, a connecting neck, and a lectin-like domain at the C terminus whose integrity is critically required for its binding activity (Sawamura T, et to the., Nature. 386; 73-77; Shi X, et al., J Cell Sci. 114; 1273-1282). LOX-1 is expressed in endothelial cells, macrophages, smooth muscle cells and platelets. Furthermore, LOX-1 is present in atheroma derived cells and is overexpressed in humans and animal atherosclerotic lesions (Kataoka H, et al., Circulation. 99; 3110-3117).

On the basis of the above scientific evidences the authors of the present invention have reported a statistically significant association between polymorphisms in the *OLR1* gene and myocardial infarction susceptibility (Mango R, et al., J Med Genet. 40; 933-936).

In the light of the above it is evident the need to have new rapid and not invasive methods for risk assessment of cardiovascular diseases, such as for instance, myocardial infarction, cerebrovascular insufficiency and peripheral vascular disease. It is also desirable to be able to have new therapeutic targets for the treatment of the aforementioned cardiovascular diseases for example through the inhibition of the formation, progression and destabilization of the atherosclerotic plaque.

The authors of the present invention have now identified and characterized a splicing isoform of the OLR1/LOX-1 gene, encoding for a protein named LOXIN that is shown to be protective against the risk to develop cardiovascular diseases such as myocardial infarction.

Through functional studies by real-time PCR, cloning and western blot analysis the authors have succeeded in identifying a new protein isoform of the LOX-1 receptor, named LOXIN, that is produced for alternative splicing of the OLR1 gene. Functional studies performed on the OLR1 gene transcript have shown how the expression of the two isoforms (OLR1/LOX-1 full length and LOXIN) is modulated by the polymorphisms previously identified by the same authors and located in the introns 4,5 and 3'UTR of the OLR1 gene. In particular, subjects carrying the risk haplotype for myocardial infarction at the polimorphisms located in the linkage disequilibrium block show a decreased expression of LOXIN and an increased expression of LOX-1 at mRNA and protein level.

Accordingly the authors have set also a rapid, economic and reliable method for the quantification of LOXIN and LOX-1 transcripts using Real-Time PCR (ABI 7000 device) starting from peripheral blood for the prediction of the risk of cardiovascular diseases, such as for instance, myocardial infarction, cerebrovascular insufficiency and peripheral vascular disease.

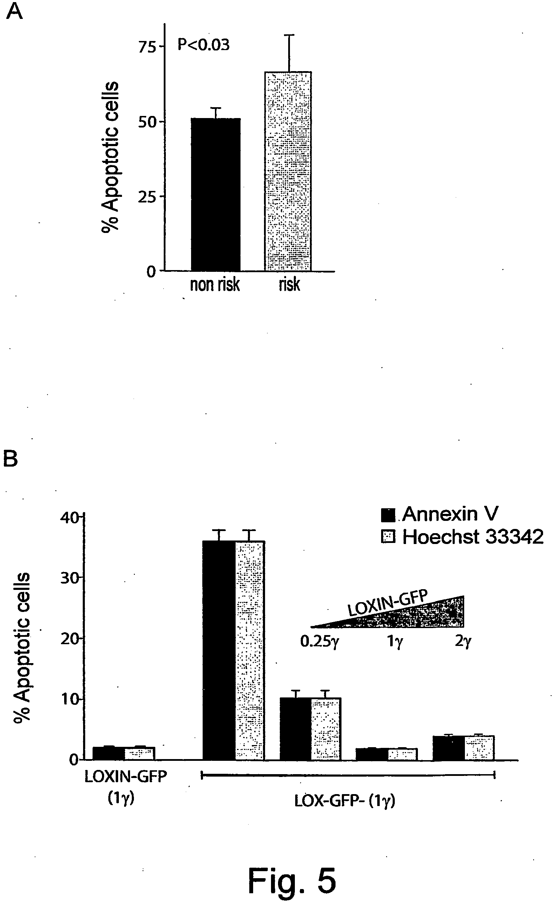

Besides as shown by flow cytometry and immunofluorescence experiments, the new identified LOXIN isoform is able to reduce OxLDL induced cytotoxicity reducing apoptosis degree by 40%. Since the apoptosis is a key step in atherosclerotic plaque destabilization, the experimental evidence of the reduction of the apoptotic index after addition of LOXIN in cell cultures treated with OxLDL or transfected with LOX-1, addresses LOXIN as possible molecular marker of plaque instability and molecular stabilizer of the atherosclerotic plaque as therapeutic implication. Such data are supported by *in vivo* experiments carried out on subjects with myocardial infarction that have shown how the macrophages of such patients, expressing a smaller quantity of LOXIN, are more susceptible to the apoptotic damage oxLDL induced (Figure 5 panel A). It is therefore an object of the present invention the use of an alternative splicing isoform of the OLR1 gene, encoding for the oxidized low density lipoproteines receptor-1 (LOX-1), characterized in that exon 5 from the nucleotide 565 to the nucleotide 680 is excised in comparison to the corresponding native oligonucleotidic sequence of the human OLR1 transcript (NM 002543) or its complementary sequence, or of the vector comprising the oligonucleotidic sequence of said alternative splicing isoform or of the amino acidic sequence encoded thereby, for the preparation of an antiapoptotic medicament inhibiting the atherosclerotic plaque instability.
It is further object of the present invention, the use of an alternative splicing isoform of the OLR1 gene encoding for the oxidized low density lipoproteines receptor-1 (LOX-1), characterized in that exon 5 from the nucleotide 565 to the nucleotide 680 is excised in comparison to the corresponding native oligonucleotidic sequence of the human OLR1 transcript (NM 002543) or its complementary sequence, or of the vector comprising the oligonucleotidic sequence of said alternative splicing isoform or of the amino acidic sequence encoded thereby, as marker for the determination of the risk of cardiovascular diseases, preferably by determining the ratio between the amounts of the corresponding native isoform and said marker.

In a preferred embodiment of the present invention, the native oligonucleotidic sequence comprises the following oligonucleotidic sequence: or its complementary sequence.

According to a further embodiment of the present invention the above defined alternative splicing isoform comprises an oligonucleotidic sequence of RNA (mRNA) or of DNA (cDNA).

The aforesaid alternative splicing isoform according to the present invention, can be labelled or fused to an oligonucleotidic sequence encoding for a protein marker, preferably selected from the group consisting of luciferase, green fluorescent protein (GFP) and its variants as well as fluorescent proteins of various type, epitopes of various type c-myc, tag recognized by monoclonal or polyclonal antibodies, fluorescent dyes, biotin.

Preferably, said amino acidic sequence includes the following amino acidic sequence:

Furthermore the present invention relates to the oligonucleotidic sequence of the alternative splicing isoform encoding for the above mentioned amino acidic sequence.

More preferably, said cardiovascular diseases are selected from the group consisting of atherosclerosis, myocardial infarction, coronary atherosclerotic disease, cerebral stroke, ischemia, acute and chronic peripheral vascular diseases.

Furthermore, the present invention refers to a method for the determination of the risk of cardiovascular diseases, preferably selected from the group consisting of atherosclerosis, myocardial infarction, coronary atherosclerotic disease, cerebral stroke, ischemia, acute and chronic peripheral vascular diseases, comprising the following steps:
a) quantitative detection of m-RNA levels of native LOX-1 and the alternative splicing isoform according to the invention in a biological sample, preferably in the peripheral blood;
b) determination of the value of the ratio R LOX-1 transcript/alternative splicing isoform transcript as defined in claims 2-9 and of the relative risk according to the following values:

| | |
|---|---|
| Low risk | R < 0,6 |
| Intermediary risk | 0,7 < R < 0,8 |
| High risk | R > 0,9. |

Preferably, the detection of step a) is carried out by Real-Time PCR and at least one oligonucleotidic probe and at least one pair of specific primers for native LOX-1 and/or of the alternative splicing isoform mRNAs according to the present invention. According to a preferred embodiment of the method of the present invention the detection is carried out by two oligonucleotidic probes and two pairs of primers able to selectively quantify the two isoforms: LOX-1 and the alternative splicing isoform LOXIN, said probes comprising the following oligonucleotidic sequences:
i) Probe LOX-1 5' - FAM-AGCTGATCTGGACTT-3' (SEQ ID NO:3);
ii) Probe LOXIN 5' - VIC-CAGCTGATCTGATTT-3'(SEQ ID NO:4);
said pairs of primers comprising:
iii) Primer LOX-1 Fw 5' - TGCCAAGTTGCTGAAAATTAATAGC-3'(SEQ ID NO:5);
vi) Primer LOX-1 Rv 5' - AACTGGAATAGGAAATTGCTTGCT-3'(SEQ ID NO:6);
v) Primer LOXIN Fw 5' - TGCCAAGTTGCTGAAAATTAATAGC-3'(SEQ ID NO:7);
vi) Primer LOXIN Rv 5'- TGAAGGGTATGTCTGGGAGACA-3'(SEQ ID NO:8).

It is an object of the present invention a diagnostic kit comprising the oligonucleotidic probes and the specific primers for the native LOX-1 isoform and for the alternative splicing LOXIN isoform according to the invention for the prediction of the risk of cardiovascular diseases, wherein said probes comprise the following oligonucleotidic sequences:
i) Probe LOX-1 5'- FAM-AGCTGATCTGGACTT-3'(SEQ ID NO:3);
ii) Probe LOXIN 5'- VIC-CAGCTGATCTGATTT-3'(SEQ ID NO:4);
and said pairs of primer comprise the following oligonucleotidic sequences:
iii) Primer LOX-1 Fw 5'- TGCCAAGTTGCTGAAAATTAATAGC-3'(SEQ ID NO:5);
iv) Primer LOX-1 Rv 5' - AACTGGAATAGGAAATTGCTTGCT-3'(SEQ ID NO:6);
v) Primer LOXIN Fw 5'- TGCCAAGTTGCTGAAAATTAATAGC-3'(SEQ ID NO:7);
vi) Primer LOXIN Rv 5'- TGAAGGGTATGTCTGGGAGACA-3'(SEQ ID NO:8).

Preferably, said cardiovascular diseases are selected from the group consisting in atherosclerosis, myocardial infarction, coronary atherosclerotic disease, cerebral stroke, ischemia, acute and chronic peripheral vascular disease.

The present invention also refers to the use of a probe for the in vitro detection of the alternative splicing isoform LOXIN according to the invention for the prediction of the risk of cardiovascular deseases, said probe comprising the following nucleotidic sequence:
5'- CAGCTGATCTGATTT-3'(SEQ ID NO:4). Preferably, said probes are labelled with a substance selected from the group consisting of fluorophores and radioisotopes, and more preferably said fluorophore is 6-carboxyl fluorescein.
Finally, the present invention relates to a pharmaceutical composition comprising the alternative splicing isoform or the amino acidic sequence or the vector as above defined, as active principle along with one or more pharmaceutically acceptable adjuvants and/or excipients. Said amino acidic sequence comprises the following amino acidic sequence:
MTFDDLKIQT VKDQPDEKSN GKKAKGLQFL YSPWWCLAAA TLGVLCLGLV VTIMVLGMQL SQVSDLLTQE QANLTHQKKK LEGQISARQQ AEEASQESEN ELKEMIETLA RKLNEKSKEQ MELHHQNLNL QETLKRVANC SAPCPQDWIW HGENCYLFSS GSFNWEKSQE KCLSLDAKLL KINSTADLF (SEQ ID NO:2). The oligonucleotidic sequence of the alternative splicing isoform encodes for the amino acidic sequence as defined above.

The present invention will be now described, for illustrative but not limitative purposes, according to its preferred embodiments, with particular reference to the figures of the enclosed drawings and examples, wherein:

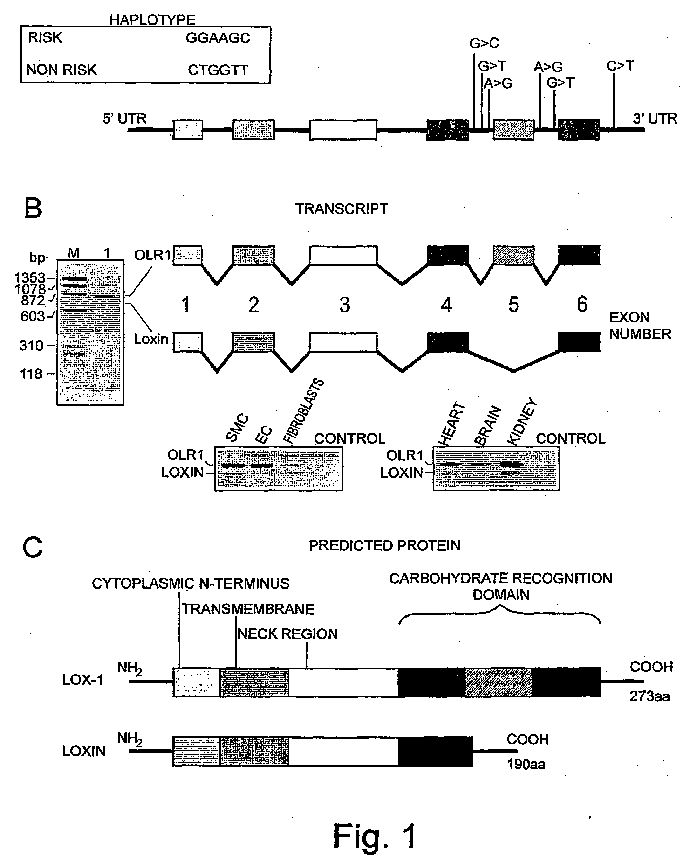

figure 1, panel A, shows genomic organization of the OLR1 gene whose exon-intron structure is shown along with SNPs position in the first part of 3' non coding region, in complete linkage disequilibrum (LD); the side box represents the "risk" and "non-risk" haplotype at the LD block; panel B shows identification of alternatively spliced forms of OLR1 gene and expression pattern in different cell types and tissues as indicated; panel C shows a predicted protein schematic representation,

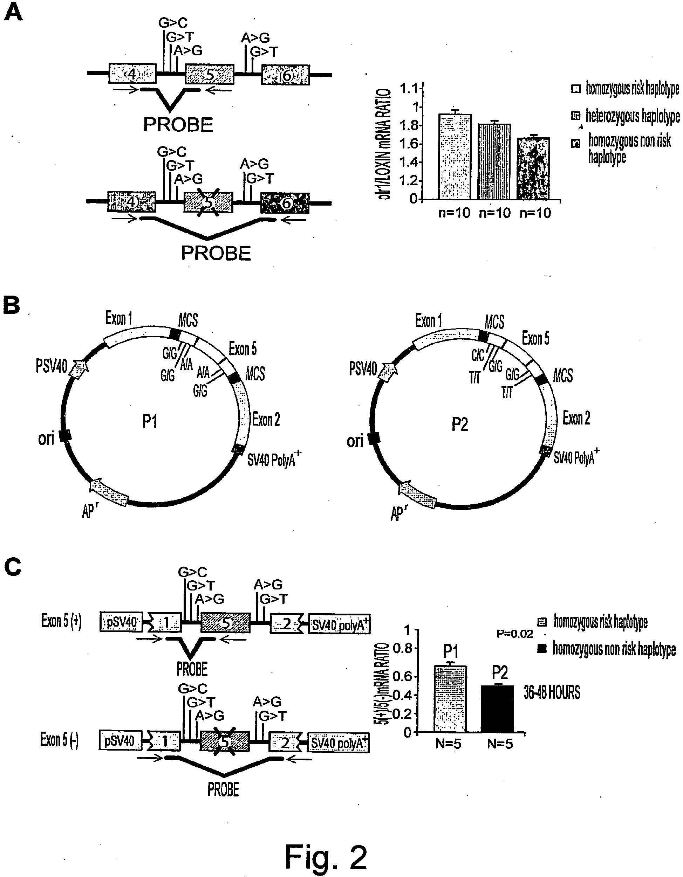

figure 2 shows how SNPs located in the LD block (linkage disequilibrum) modulate the levels of the OLR1 mRNA isoforms; panel A shows quantification of the OLR1 and LOXIN transcripts using quantitative real-time PCR; graphs show relative amounts of the two expressed isoform quantitative as ratio between OLR1 isoform (containing exon 5) and LOXIN isoform (lacking exon 5); panel B shows the map of the two minigene plasmids P1 and P2 containing the genomic sequence homozygous for the "risk" haplotype and the "non-risk" haplotype, respectively; panel C shows quantification of the P1 and P2 minigenes expression; graphs show the ratio between the transcript containing exon 5 and transcript lacking exon 5;

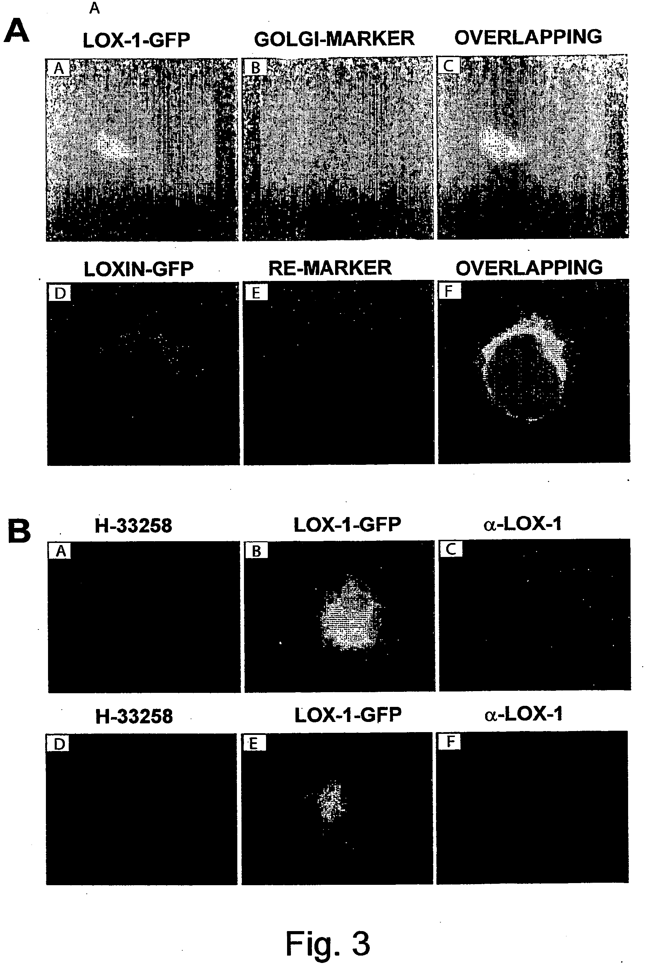

figure 3 shows subcellular distribution of LOX-1 and LOXIN isoforms; panel A and panel D show COS-7 cells transfected with LOX-1-GFP and LOXIN-GFP respectively; panel B and panel E show the same cells costained with antibodies against the Golgi 58K protein and calnexin; panels C and F represent the merged images; in panel B, unpermeabilised transfected COS-7 cells were immunostained with anti-LOX-1 antibodies;

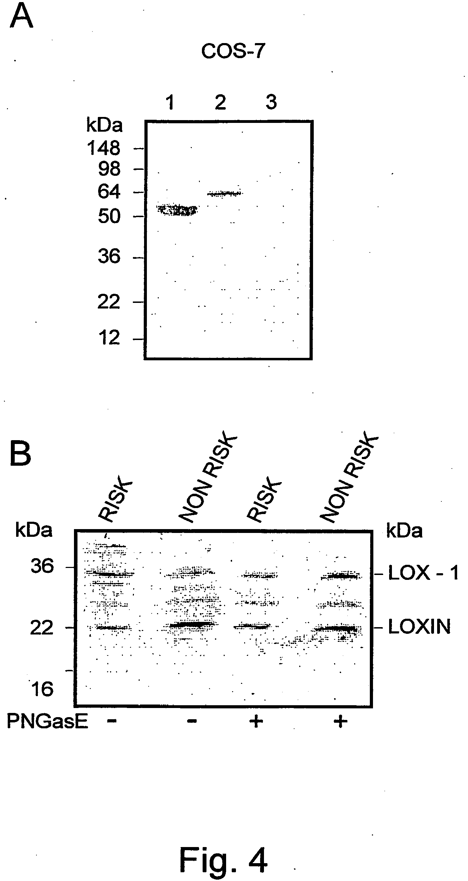

figure 4 shows intracellular expression of LOX-1 and LOXIN isoforms; panel A shows Western blot analysis of cellular lysates derived from mock-transfected (lane 3) or COS-7 cells transiently transfected with DNA encoding for LOXIN-GFP (lane 1) and LOX-1-GFP (lane 2); the two isoforms were detected with rabbit anti-LOX-1 antibodies; panel B shows Western blot analysis of cellular lysates of PBMCs derived from subjects carrying the "risk" (lanes 1 and 3) and "non-risk" (lanes 2 and 4) haplotypes incubated for 3 hours with PNGase F (1000 U), as indicated;
figure 5 shows the protective effect of LOXIN expression; panel A shows percentage of apoptotic cells in isolated monocytes-macrophages samples isolated from PBMCs of 10 subjects homozygous for the "risk" haplotype and 10 for the "non-risk" haplotype; panel B, shows COS-7 fibroblasts transiently transfected with the vector encoding for LOXIN-GFP and LOX-1-GFP alone or cotransfected with a fixed amount of LOX-1 and increasing concentration of LOXIN-GFP plasmids (as indicated); the average of the percentage of apoptotic cells in cells transfected with GFP alone (6%) was subtracted to all experimental points; the results shown represent average of three independent experiments.

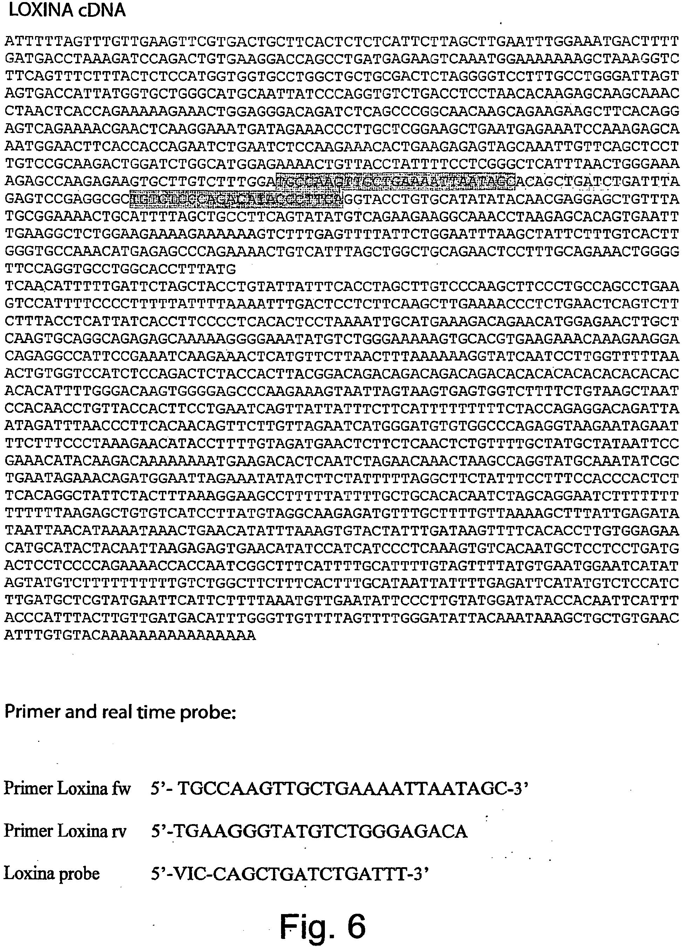

figure 6 shows the sequence of the LOXIN cDNA, Fw and Rv primers and the probe labelled with VIC fluorochrome thereof.

### EXAMPLE 1: Identification of alternative isoforms of the OLR1 gene

This study reports, firstly, the identification and characterization of a new functional isoform of the *ORL1* gene and provides a functional characterization of the genetic association between SNPs within *ORL1* gene and myocardial infarction.

It has been demonstrated that SNPs located in the linkage disequilibrium (LD) block modulate the relative abundance of the two transcripts: OLR1 (native transcript) and LOXIN (alternative splicing isoform). Both in vivo and in vitro experiments indicate that the new splice variant LOXIN is expressed at a similar level as the full-length receptor LOX-1. Macrophages from subjects carrying the "non-risk" haplotype express more LOXIN and result in fewer cells undergoing apoptosis on oxLDL induction. Macrophages play an important role in all phases of formation of atherosclerosis plaque, from the development of the fatty steak to the process that ultimately contribute to plaque rupture and myocardial infarction (Li AC, Glass CK. The macrophage foam cell as a target for therapeutic intervention. Nat Med. 8;1235-1242; Kavurma MM, Bhindi R, Lowe HC, Chesterman C, Khachigian LM. Vessel wall apoptosis and atherosclerotic plaque instability. J Thromb Haemost. 3;465-472; Tabas I). Apoptosis and plaque destabilization in atherosclerosis: the role of macrophage apoptosis induced by cholesterol. Cell Death Differ. 11;12-16). Indeed, evidence from pathological studies link apoptosis of plaque resident macrophages with rupture and thrombosis of atherosclerotic lesions and subsequent development of acute vascular complication.

In particular, it has been proposed that extensive apoptosis of macrophages occurs only at sites of plaque rupture and possibly contributes to the process of rupture and thrombosis (Kolodgie FD, Narula J, Burke AP, Haider N, Farb A, Hui-Liang Y, Smialek J, Virmani R. Localization of apoptotic macrophages at the site of plaque rupture in sudden coronary death. Am J Pathol. 157; 1259-1268). It is our finding that increased levels of LOXIN and reduced levels of apoptosis in macrophages suggest that this in turn could result in plaque stabilization by influencing the nature of the vulnerable plaque. The study shows that different cellular localization of the two isoforms by mean of immunofluorescence studies. In cell lines transfected with the LOXIN-GFP construct we show that LOXIN isoform displays no surface expression in 90% of transfected cells. This is attributable to retention of LOXIN in the endoplasmic reticulum ER and accumulation of the protein in the perinuclear region. Regulation of cellular trafficking of LOX-1 receptor to the plasma membrane may provide an essential mechanism for the control of its function in vivo. In this context, different splice variant isoforms in the C-terminal domain have been reported for other receptors, such as glutamate receptors. In this case, the domain has been shown to be the site of interactions of proteins involved in the trafficking and stabilization of glutamate receptors in the synaptic membrane and, in turn, to play a role in synaptic strength and plasticity (Barry MF, Ziff EB. Receptor trafficking and the plasticity of excitatory synapses. Curr Opin Neurobiol. 12;279-286; Carroll RC, Zukin RS. NMDA-receptor trafficking and targeting: implications for synaptic transmission and plasticity. Trends Neurosci. 25; 571-577).

Whether LOXIN forms heteromeric receptors with LOX-1 and regulates its intracellular traffic in vivo is under study.

LOX-1 receptor, when ectopically expressed in fibroblasts, is toxic and results in a high percentage of cells undergoing apoptosis. We demonstrate here that LOXIN molecules, when coexpressed with LOX-1 receptors, have a protective role and are able to rescue the apoptotic phenotype. The mechanism by which LOXIN exerts its protective role is not known but we propose different hypotheses. First, LOXIN may act on LOX-1 receptors by forming inactive heterodimers. Because the LOXIN splice variant lacks exon 5, it misses the binding region of ox-LDL. By increasing the intracellular LOXIN relative amount, the number of functional receptors able to bind oxLDL may be reduced. Because oxLDL induces apoptosis in macrophages, thereby reducing oxLDL binding sites, fewer cells will go through apoptosis. Secondly, LOXIN may exert its action on the transport of LOX-1 receptors toward the cellular membrane, blocking them into the ER and downregulating their membrane expression. Thirdly, the LOXIN itself may even have an antiapoptotic activity. These different hypotheses warrant further studies.

It is important to note that the expression level of both isoforms in vivo is similar. Subjects homozygous for "nonrisk" haplotype show a small increase in LOXIN expression compared with homozygous for "risk" haplotype. In vitro experiments confirmed this fine balance between the 2 isoforms. A small amount of LOXIN is sufficient to rescue the lethal phenotype induced by LOX-1 polymorphism localized in the LD block of *OLR1* gene, with their functional role, may represent an important genetic risk factor for prediction of susceptibility to myocardial infarction. Moreover LOXIN could represent a new target for prevention of progression and destabilization of atheroslerotic plaque. Therefore, new research or therapeutic strategies favoring increasing expression of LOXIN could be effective for preventing plaque instability.

### MATERIALS AND METHOD

### RT-PCR experiments

Total RNA was purified from monocyte-derived macrophages and COS-7 cells using RNeasy Mini Purification kit (Qiagen). Poly A⁺ RNA were purified using the Oligotex™ mRNA Mini Kit (Qiagen). Reverse transcription was performed with a High-Capacity cDNA
Archive Kit (Applied Biosystems). RT products were amplified using forward OLR1-FW primer (5'-TGTTGAAGTTCGTGACTGCTT-3'(SEQ ID NO:9)) and reverse OLR1-RW primer (5'-TTCTGCAGCCAGCTAAATGA-3'(SEQ ID NO:10)), and then subcloned using a TA cloning kit (Invitrogen). *DNA constructs*
Minigene: To amplify the genomic sequence surrounding the alternatively spliced exon 5 of *OLR1* gene, we used the primer pair *OLR1*-*Xno*lF (5'-ACA GTC *CTC GAG* GTG AGT GTT CAT GGA TAT TTG-3'(SEQ ID NO:11)) and OLR1-*Eco*RIR (5'TGT GTG GAT ATC CTG CAG GTA GGA AAA ACA AAA-3'(SEQ ID NO:12)). We used human genomic DNA homozygous with respect to "risk" or "non-risk" haplotype at LD block of OLR1 gene as the PCR template. The PCR products were cloned into pSPL3 vector (Invitrogen) and sequenced using the primer *OLR1*SEQF (5'-GTT TCC TAT TCT TTG CTG AAC-3'(SEQ ID NO:13)) and *OLR1*SEQR (5'-GTG GGG AGT AAT GTT TCT GAG-3'(SEQ ID NO:14)). Recombinant protein: To generate LOX-1-GFP and LOXIN-GFP constructs, coding sequences of *OLR1* gene and the splicing variant LOXIN were PCR-amplified from cDNA, which was derived from the human heart poly A⁺ RNA (Clontech) using selected oligos. For the ampl ification of OLR1, primers F1 (5'CCG*CTCGAG*ATGACTTTTGATGACCTAAAG 3'(SEQ ID NO:15)) and F2 (5' CGCGGATCCT GTGCTCTTAGGTTTGC 3'(SEQ ID NO:16)) were used. For the amplification of LOXIN, primers F1 and F3 (5'CGC*GGATCC*ATCA GATCAGCTGTGCTATT 3'(SEQ ID NO:17)) were used. The PCR products were cloned into the XhoI/BamHI-digested pEGFP-N1 vector (Clontech) and sequenced using CEQ2000 (Beckman-Coultard).

### Quantitative PCR real-time

Real-time RT-PCR was performed on a TaqMAN ABI 7000 Sequence Detection System (Applied Biosystems). By using the Primer Express 2.0 software (Applied Biosystems), we designed primers and MGB probes for specific quantitative analysis of native transcript (OLR1) and the alternatively spliced isoforms. The sequences of the primers and of the MGB probes are the followings:
i) Probe LOX-1 5'- FAM-AGCTGATCTGGACTT-3'(SEQ ID NO:3);
ii) Probe LOXIN 5'- VIC-CAGCTGATCTGATTT-3' (SEQ ID NO:4;
and said pairs of primer comprising the following oligonucleotide sequences:
iii) Primer LOX-1 Fw 5'-TGCCAAGTTGCTGAAAATTAATAGC-3' (SEQ ID NO:5);
iv) Primer LOX-1 Rv 5' - AACTGGAATAGGAAATTGCTTGCT-3'(SEQ ID NO:6);
v) Primer LOXIN Fw 5' - TGCCAAGTTGCTGAAAATTAATAGC-3'(SEQ ID NO:7);
vi) Primer LOXIN Rv 5' - TGAAGGGTATGTCTGGGAGACA-3'(SEQ ID NO:8).

Commercially available predeveloped TaqMan endogenous reference GAPDH gene (Applied Biosystems) was used to normalize the amount of cDNA added per sample. A comparative CT method was used to determine relative quantification of RNA expression. All PCR reactions were performed in triplicate.

Results are represented as a ratio between expression levels of native transcript (OLR1) normalized for the control gene (GAPDH) and the expression levels of alternative splicing isoform (LOXIN) normalized for the control gene (GAPDH).

### Cell Culture and Transfection

Human monocytes were isolated from peripheral blood mononuclear cells (PBMCs) of subjects homozygous for the "risk" and "non-risk" haplotype. We promoted their transition to macrophages in vitro as previously described, (Sawamura T, et al., Nature, 386; 73-77). Differentiation was determined by flow cytometry using anti-CD36 FITC monoclonal antibody (cell purity >95%). COS-7 fibroblasts were grown in Dulbecco's modified Eagle's medium (DMEM; Invitrogen) supplemented with 10% fetal bovine serum (FBS). Transient transfection of COS-7 was performed using the Nucleofector kit (Amexa Biosytems). 1 µg of plasmid DNA was added to 5x10⁵ COS-7 suspended in 100 µL of human dermal fibroblast Nucleofector solution (Amexa Biosytems). A-24 program was selected for a high density of transfection. All subjects gave informed consent, and the study protocol was approved by the Tor Vergata University Ethics Committee.

### Evaluation of Apoptosis

Differentiated macrophages were cultured without serum for 15 hours and then incubated with oxLDL (100 µg/mL; Intracel) for 8 hours before detection of apoptosis. Apoptosis was evaluated by multiparameter flow cytometry using a method that distinguishes nuclei from apoptotic, necrotic, or viable lymphoid cells (Matteucci C, et al., Cytometry. 35;145-153). Isolated nuclei were analyzed by fluorescence and by forward- and side-angle scatter multiparameter analysis using a FACSscan Flowcytometer(Becton Dikinson). A minimum of 5000 events was collected for each sample. Apoptotic fibroblasts were visualized by staining with the blue fluorescent dye Hoechst 33342 (Sigma) and phosphatidylserine assay as described previously (Filesi I, et to the.,Journal of Cell Science. 115;1803-1813). Annexin V and Hoechst 33342 positive cells were counted from cells transfected with GFP (green fluorescent protein), LOX-1-GFP, and LOXIN-GFP recombinant proteins.

### Immunofluorescence Staining

Immunofluorescence was performed as described. (Heinloth A, et al., Atherosclerosis. 162; 93-101). Affinity purified anti-rabbit LOX-1 (Santa Cruz), goat anti-calnexin (Santa Cruz), and mouse anti-Golgi 58K (Sigma) protein were used as primary antibodies. Texas Red goat anti-rabbit IgG(Calbiochem), Texas Red goat anti-mouse IgG (Calbiochem), and Rhodamine Red-Xconjugated affinipure rabbit anti-goat IgG (Jackson Immunoreasearch) were used as secondary antibodies. Hoechst 33342 dye was used at 1 µg/mL. Samples were examined with a DMRA Leica fluorescence microscope equipped with CCD camera. Acquired images were deconvolved using Leica Q-fluoro software and processed using Adobe Photoshop.

### Western Blot Analysis and Enzymatic Digestion

PBMCs and COS-7 cells were lysed for 30 minutes in ice-cold cell extraction buffer (EB) (100 mmol/L NaCl, 10 mmol/L EDTA, 0.5% Nonidet P-40, 0.5% sodium deoxycholate, 10 mmol/L Tris-HCl, pH 7.4, 1 mmol/L PMSF, 10 µg/mL pepstatin A, 10 µg/mL leupeptin and 0.3 µM/L aprotinin). Nuclei and large debris were removed by centrifugation at 290g for 10 minutes at 4°C. The supernatants were then precipitated with 5 volumes of MeOH at -20°C for 2 hours. After centrifugation (16000 g, 10 minutes, 4°C), protein pellets were dissolved in 4 sample buffer (500 mmol/L Tris-HCl, pH 6.8, 4% SDS, 20% glycerol, 40 mmol/L DTT and 0.02% bromophenol blue) and heated at 95°C for 5 minutes.

Blots were probed with rabbit anti-LOX-1 antibody (Santa Cruz).

Immunoreactive bands were detected with sheep anti-rabbit IgG horseradish peroxidase (Amersham) and visualized by ECL (Sigma). Enzymatic deglycosylation was performed as previously described.(Cardinal To, et to the., Methods. 34;171-178).

### Statistic analysis

All statistical analyses were performed using SPSS version 13 software. Data are presented as means ±1 standard deviation (SD). Normal distribution of continuous variables has been verified by Kolmogorov-Smirnov with Lillefors correction. Differences in means of continuous variables were analyzed by impaired *t* test or ANOVA as needed. Bonferroni correction was used for multiple comparison.

### RESULTS

### Identification of an Alternatively Spliced Forms of

### OLR1 Gene

We started from total or poly(A)+ RNA of human monocytes derived macrophages and subjected it to reverse transcription to test the potential effect of the SNPs located in the LD block on RNA splicing of the human *OLR1* gene. We performed PCR amplification using primers located in the 5'UTR and 3'UTR of the *OLR1* gene and sequenced the amplified fragments. This analysis identified a reproducible pattern of alternative splicing around exon 5 (from nucleotide 565 to nucleotide 680 of the sequence of OLR transcript). In particular we identified two *OLR1* transcripts in both RNA fractions. One of these products corresponded to the full-length transcript (*OLR1*), while the other lacked exon 5; we named it LOXIN (Figure 1 B). The newly spliced mRNA has a stop codon in the open reading frame that leads to a premature termination of the translation product and generates a predicted protein that lacks 2/3 of the lectin-like domain (Figure 1 C). Both isoforms were detected in several cell types (endothelial cells, fibroblasts, and smooth muscle cells), and tissues (heart, kidney, and brain), suggesting that they reflect a physiological pattern of expression of the *OLR1* gene (Figure 1 B)

### SNPs Located in the LD Block Modulate the Levels of mRNA Isoforms

To find in vivo evidence that SNPs located in the LD block modulate the level of the two mRNA isoforms, we performed an isoform-specific real-time PCR starting from total RNA of human monocyte-derived macrophages of selected patients carrying the "risk" and "non-risk" haplotype at *OLR1* gene. By this analysis we noted a marked difference in the mRNA ratio (*OLRI*/LOXIN) according to the haplotype. In particular, the *OLR1*/LOXIN mRNA ratio was 33% higher in human monocyte-derived macrophages of subjects homozygous for the "risk" haplotype compared with homozygous for the "non-risk" haplotype (Figure 2A).

The ratio R of OLR1/LOXIN mRNAs has been correlated to the risk of myocardial infarction:

| | |
|---|---|
| Low risk | R < 0,6 |
| Intermediate risk | R 0,7-0,8 |
| High risk | R > 0,9. |

The finding that the relative amount of the LOXIN transcript is significantly greater in subjects carrying the "non-risk" haplotype strongly suggests a negative link between levels of LOXIN mRNA and the incidence of myocardial infarction in humans.

To further confirm the regulatory role of the intronic polymorphism, we extended these studies by constructing minigenes carrying the "risk" and "non-risk" haplotypes with genomic sequences containing intron 4, exon 5, and intron 5 (Figure 2B). These constructs were transfected in COS-7 fibroblasts and the ratio of unspliced (exon 5+) to spliced (exon 5-) transcript was analyzed by real time isoform specific PCR. As can be seen in Figure 2C, the ratio of the unspliced form (5+) vs the spliced form (5-) was 27% higher in RNA extracted from cells transfected with minigenes carrying the "risk" haplotype. These in vitro experiments not only suggest that the relative abundance of the two isoforms is modulated by the intronic SNPs mapping within the LD block, but also confirm the previously described in vivo results (Figure 2A).

### Subcellular and Membrane Distribution of LOX and the LOXIN Splice Variant

To investigate the cellular localization of the natiuve transcript (OLR1) and the splice variant, we constructed two plasmids that allowed the efficient expression of LOX-1 and LOXIN in mammalian cells fused, C terminally, to the green fluorescent protein (GFP). Immunofluorescence analysis of transfected COS-7 fibroblasts revealed that the intracellular expression of the full-length LOX-1-GFP causes a cell-lethal phenotype. Many transfected cells are roundly shaped and tend to detach from the dish. On the contrary, LOXIN-GFP isoform is efficiently expressed, and its expression does not result in cytotoxicity. Notwithstanding the toxic effect, many LOX-1-GFP transfected cells retain normal morphology, and this has allowed us to study its intracellular distribution.

As shown in Figure 4A, (panels A-C), LOX-1 distributed in the ER and in the Golgi apparatus. At 24 hours after transfection, LOX-1 colocalized almost exclusively with the Golgi 58K protein, indicating that LOX-1 receptor in the initial phase of synthesis traffics along the secretory pathway. In contrast, LOXIN-GFP was not detectable in the Golgi patches of permeabilized COS-7 cells. In these cells we observed a more widespread staining, characteristic of typical ER distribution. In 40% to 50% of cells transfected with LOXIN-GFP, an accumulation of fluorescence in the perinuclear area was also detected (Figure 4A, panels D-F). The two GFP-tagged isoforms were also labeled for surface receptors in live cells (Figure 3B and 4). At 24 hours after transfection, over 90% of cells expressing LOX-1-GFP showed a typical punctuate plasma membrane-associated fluorescence (Figure 4B, panel C). Remarkably, less than 10% of COS-7 cells transfected with LOXIN-GFP showed surface staining (Figure 3B, panel F). The very low expression of LOXIN at the plasma membrane demonstrates that truncation of the C-terminal portion in LOXIN protein leads to a profound effect on cellular trafficking of the protein to the plasma membrane.

The expression level of the two isoforms in transfected fibroblasts was also confirmed in Western blot. A single band corresponding to LOX-1-GFP and LOXIN-GFP fusion proteins were detected (Figure 4A, lanes 1 and 2). The molecular weight of the bands corresponded to the predicted molecular weight of nonglycosylated proteins. The LOX-1-GFP band was, however, much fainter, probably because of the previously mentioned cytotoxic effect of this construct (Figure 4A, lane 2).

### In Vivo Expression of LOX 1 and LOXIN Isoforms

To verify whether LOXIN transcript is indeed translated in vivo and to analyze the level of its expression, we used Western blot to examine the relative amounts of the two isoforms in PBMCs derived from selected subjects with different haplotypes. As shown in Figure 4B, immunoreaction of cell lysates showed 2 major bands of 34 and 22 kDa, corresponding to the predicted molecular weight of the two isoforms. Interestingly, we noticed a relative increase in the amount of LOXIN in cells derived from subjects homozygous for the "non-risk" haplotype (Figure 4B, lanes 2 and 4). Removal of N-linked glycans by PNGase digestion (Figure 4B, lanes 3 and 4) resulted in the disappearance of few faint bands, indicating that the two bands, 34 and 22 kDa, correspond to the unglycosylated LOX-1 and LOXIN proteins. In many gels, including the one shown in Figure 4B, a third band of 26 kDa was also observed. This band may represent a degradation product of the LOX-1 protein that we are currently studying.

### In Vivo Proapoptotic Effect of LOX-1 and Rescue By LOXIN

We considered that an altered balance between the 2 isoforms could be related to the increased susceptibility to apoptosis.

To test this notion, we analyzed oxLDL-induced apoptosis in a monocytes/macrophages in vivo assay (Heinloth A, et al., Atherosclerosi, 162; 93-101). Human monocytes were isolated from buffy coats of healthy donors carrying the "risk" and "non-risk" haplotype at LD block of *OLR1* gene. After Ficoll gradient centrifugation, monocytes were separated to induce differentiation in macrophages. 100 µg/mL of oxLDL was added to these cells to induce apoptosis. Remarkably, flow cytometric analysis of macrophages derived from subjects homozygous for the "non-risk" haplotype showed a 24% reduction in apoptotic cell number compared with the homozygous for the "risk" haplotype when treated with the oxLDL (Figure 5A). Our data suggest that increased levels of LOXIN in macrophages may relate to reduced level of apoptosis.

Because the two isoforms are physiologically coexpressed in PBMCs with a different balance related to the haplotype, and increased LOXIN levels relate to the "non-risk" haplotype, we explored the possibility that LOXIN isoform may have a protective effect versus apoptosis. To test this hypothesis, we transfected COS-7 fibroblasts with DNA encoding for LOXIN-GFP and LOX-1-GFP alone or cotransfected with a fixed amount of LOX-1 and increasing concentration of LOXIN-GFP plasmids (as indicated in Figure 5B). The phenotype of transfected cells was analyzed using 2 markers of apoptosis. First, we used Annexin V, which detects alteration at the level of the plasma membrane. Next, we examined the uptake of blue-fluorescent Hoechst 33342 dye, which stains the condensed chromatin of apoptotic cells more brightly than the chromatin of nonapoptotic cells. On the basis of the combined staining patterns of these dyes, we were able to distinguish between normal, apoptotic, and dead cells.

As above mentioned, cells expressing the LOX-1-GFP fusion protein did not thrive, with many of the cells exhibiting cell shrinkage, which is a feature of apoptosis. Thus, LOX-1-GFP alone resulted in 36% of cells developing apoptosis. In contrast, LOXIN expression was not toxic and the number of apoptotic cells was comparable to the mock-transfected cells.

Interestingly, the coexpression of LOXIN-GFP resulted in a complete dose-dependent rescue of the LOX-1-GFP-induced phenotype. It is worth noting that a very low dose of LOXIN, corresponding to a ratio of 1:4 with LOX-1, resulted in a 72% reduction in the number of apoptotic cells, and a 1:1 ratio of the 2 plasmids used for transfection completely prevented the phenotype. This finding suggests that small differences in LOX-1/LOXIN balance and, especially, a small increase in LOXIN expression, may have a very profound effect on the cytotoxic phenotype also in vivo.

## Claims

1. Use of an alternative splicing isoform of the OLR1 gene encoding for the oxidized low density lipoproteines receptor-1 (LOX-1), **characterized in that** exon 5 from the nucleotide 565 to the nucleotide 680 is excised in comparison to the corresponding native oligonucleotidic sequence of the human OLR1 transcript (NM 002543) or its complementary sequence, or of the vector comprising the oligonucleotidic sequence of said alternative splicing isoform or of the amino acidic sequence encoded thereby,
for the preparation of an antiapoptotic medicament inhibiting the atherosclerotic plaque instability.

2. Use of an alternative splicing isoform of the OLR1 gene encoding for the oxidized low density lipoproteines receptor-1 (LOX-1), **characterized in that** exon 5 from the nucleotide 565 to the nucleotide 680 is excised in comparison to the corresponding native oligonucleotidic sequence of the human OLR1 transcript (NM 002543) or its complementary sequence, or of the vector comprising the oligonucleotidic sequence of said alternative splicing isoform or of the amino acidic sequence encoded thereby,
as marker for the determination of the risk of cardiovascular diseases, preferably by determining the ratio between the amounts of the corresponding native isoform and said marker.

3. Use according to claim 2, wherein said cardiovascular diseases are selected from the group consisting in atherosclerosis, myocardial infarction, atherosclerotic coronary disease, cerebral stroke, ischemia, acute and chronic peripheral vascular diseases.

4. Use according to anyone of the claims 1 to 3, wherein said native oligonucleotidic sequence comprises the following oligonucleotidic sequence:
atgacttttg atgacctaaa gatccagact gtgaaggacc agcctgatga gaagtcaaat ggaaaaaaag ctaaaggtct tcagtttctt tactctccat ggtggtgcct ggctgctgcg actctagggg tcctttgcct gggattagta gtgaccatta tggtgctggg catgcaatta tcccaggtgt ctgacctcct aacacaagag caagcaaacc taactcacca gaaaaagaaa ctggagggac agatctcagc ccggcaacaa gcagaagaag cttcacagga gtcagaaaac gaactcaagg aaatgataga aacccttgct cggaagctga atgagaaatc caaagagcaa atggaacttc accaccagaa tctgaatctc caagaaacac tgaagagagt agcaaattgt tcagctcctt gtccgcaaga ctggatctgg catggagaaa actgttacct attttcctcg ggctcattta actgggaaaa cagccaagag aagtgcttgt ctttggatgc caagttgctg aaaattaata gcacagctga tctggacttc atccagcaag caatttccta ttccagtttt ccattctgga tggggctgtc tcggaggaac cccagctacc catggctctg ggaggacggt tctcctttga tgccccactt atttagagtc cgaggcgctg tctcccagac atacccttca ggtacctgtg catatataca acgaggagct gtttatgcgg aaaactgcat tttagctgcc ttcagtatat gtcagaagaa ggcaaaccta agagcacagt ga (SEQ ID NO:1) or its complementary sequence.

5. Use according to anyone of the claims 1-4, wherein the oligonucleotidic sequence is RNA or DNA.

6. Use according to claim 5, said isoform being labelled or being fused with a oligonucleotidic sequence encoding for a protein marker.

7. Use according to claim 6, wherein said protein is selected from the group consisting of luciferase, green fluorescent protein (GFP) and its variants, fluorescent proteins, c-myc or tag epitopes recognized by monoclonal or polyclonal antibodies, fluorescent dyes, biotin.

8. Use according to anyone of the claims 1-7, wherein said amino acidic sequence comprises the following amino acidic sequence:

9. Use according to anyone of the claims 1-7, wherein the oligonucleotidic sequence of the alternative splicing isoform encodes for the amino acidic sequence as defined in claim 8.

10. Method for the determination of the risk of cardiovascular diseases comprising the following steps:
a) quantitative detection of mRNAs levels of LOX-1 and the alternative splicing isoform as defined according to anyone of the claims 2-9 in a biological sample;
b) determination of the value of the ratio R LOX-1 native transcript/alternative splicing isoform transcript **characterized in that** exon 5 from the nucleotide 565 to the nucleotide 680 is excised in comparison to the corresponding native oligonucleotidic sequence of the human OLR1 transcript (NM 002543) and of the relative risk on the following values basis:
| | |
|---|---|
| Low risk | R < 0,6 |
| Intermediate risk | R among 0,7-0,8 |
| High risk | R > 0,9. |

11. Method according to claim 10, wherein the detection of step a) is carried out by means of Real-Time PCR through at least one oligonucleotidic probe and at at least a pair of specific primer specific for mRNAs of native LOX-1 and/or the alternative splicing isoform as defined according to anyone of the claims 2-9.

12. Method according to claim 11, wherein the detection is carried out by two oligonucleotidic probes and two pairs of primer able to selectively quantify the two isoforms LOX-1 and the alternative splicing LOXIN isoforms, said probes comprise the following oligonucleotidic sequences:
i) Probe LOX-1 5'- FAM-AGCTGATCTGGACTT-3' (SEQ ID NO:3);
ii) Probe LOXIN 5'- VIC-CAGCTGATCTGATTT-3'(SEQ ID NO:4);
said pairs of primer comprising:
iii) Primer LOX-1 Fw 5'- TGCCAAGTTGCTGAAAATTAATAGC-3' (SEQ ID NO:5);
iv) Primer LOX-1 Rv 5'- AACTGGAATAGGAAATTGCTTGCT-3'(SEQ ID NO:6);
v) Primer LOXIN Fw 5'- TGCCAAGTTGCTGAAAATTAATAGC-3'(SEQ ID NO:7);
vi) Primer LOXIN Rv 5' - TGAAGGGTATGTCTGGGAGACA-3'(SEQ ID NO:8).

13. Method according to anyone of the claims from 10 to 12, wherein said cardiovascular diseases are selected from the group consisting in atherosclerosis, myocardial infarction, atherosclerotic coronary disease, cerebral stroke, ischemia, acute and chronic peripheral vascular diseases.

14. Method according to anyone of the claims from 10 to 13, wherein said biological sample is peripheral blood.

15. Diagnostic kit comprising oligonucleotidic probes and specific primers for the native LOX-1 isoform and for alternative splicing isoform LOXIN **characterized in that** exon 5 from the nucleotide 565 to the nucleotide 680 is excised in comparison to the corresponding native oligonucleotidic sequence of the human OLR1 transcript (NM 002543), for the prediction of the risk of cardiovascular disease, in which said probes comprise the following oligonucleotidic sequences:
i) Probe LOX-1 5'- FAM-AGCTGATCTGGACTT-3' (SEQ ID NO:3);
ii) Probe LOXIN 5'- VIC-CAGCTGATCTGATTT-3'(SEQ ID NO:4);
said pairs of primer comprise the following oligonucleotidic sequences:
iii) Primer LOX-1 Fw 5'- TGCCAAGTTGCTGAAAATTAATAGC-3' (SEQ ID NO:5);
iv) Primer LOX-1 Rv 5'- AACTGGAATAGGAAATTGCTTGCT-3'(SEQ ID NO:6);
v) Primer LOXIN Fw 5'- TGCCAAGTTGCTGAAAATTAATAGC-3'(SEQ ID NO:7);
vi) Primer LOXIN Rv 5' - TGAAGGGTATGTCTGGGAGACA-3'(SEQ ID NO:8).

16. Kit according to claim 15 , wherein said cardiovascular diseases are selected from the group consisting in atherosclerosis, myocardial infarction, atherosclerotic coronary disease, cerebral stroke, ischemia, acute and chronic peripheral vascular diseases.

17. Use of a probe for the in vitro detection of the alternative splicing isoform LOXIN **characterized in that** exon 5 from the nucleotide 565 to the nucleotide 680 is excised in comparison to the corresponding native oligonucleotidic sequence of the human OLR1 transcript (NM 002543) comprising the following nucleotidic sequence:
5'- CAGCTGATCTGATTT-3'(SEQ ID NO:4).

18. Use according to claim 17, wherein said probe is labelled with a substance selected from the group consisting in fluorophore, radioisotope, luminescent substance.

19. Pharmaceutical composition comprising the alternative splicing isoform as defined according to anyone of the claims 1-5, or the vector comprising the oligonucleotidic sequence of said alternative splicing isoform or the amino acidic sequence encoded thereby, as active principle along with one or more pharmacologically acceptable adjuvants and/or excipients.

20. Pharmaceutical composition according to claim 19, wherein said amino acidic sequence comprises the following amino acidic sequence:

21. Pharmaceutical composition according to claim 19, wherein the oligonucleotidic sequence of the alternative splicing isoform encodes for the amino acidic sequence as defined in claim 20.

## Patentansprüche

1. Verwendung einer alternativen Splice-Isoform des OLR1-Gens, codierend für den Rezeptor-1 für oxidierte Low-Density-Lipoproteine (LOX-1), **dadurch gekennzeichnet, dass** das Exon 5 von Nukleotid 565 bis Nukleotid 680 im Vergleich zu der entsprechenden nativen Oligonukleotidsequenz des Human-OLR1-Transkripts (NM 002543) oder ihrer komplementären Sequenz ausgeschnitten ist, oder des Vektors, welcher die Oligonukleotidsequenz der alternativen Splice-Isoform umfasst, oder der davon codierten Aminosäuresequenz
zur Herstellung eines anti-apoptotischen Medikaments, welches der Instabilität von Arterioskleroseplaques entgegenwirkt.

2. Verwendung einer alternativen Splice-Isoform des OLR1-Gens, codierend für den Rezeptor-1 für oxidierte Low-Density-Lipoproteine (LOX-1), **dadurch gekennzeichnet, dass** das Exon 5 von Nukleotid 565 bis Nukleotid 680 im Vergleich zu der entsprechenden nativen Oligonukleotidsequenz des Human-OLR1-Transkripts (NM 002543) oder ihrer komplementären Sequenz ausgeschnitten ist, oder des Vektors, welcher die Oligonukleotidsequenz der alternativen Splice-Isoform umfasst, oder der davon codierten Aminosäuresequenz,
als Marker für die Bestimmung des Risikos für kardiovaskuläre Erkrankungen, vorzugsweise durch Bestimmung des Verhältnisses zwischen den Mengen der entsprechenden nativen Isoform und des Markers.

3. Verwendung nach Anspruch 2, wobei die kardiovaskulären Erkrankungen aus der Gruppe, die aus Arteriosklerose, Myokard-Infarkt, arteriosklerotischer Koronarerkrankung, Gehirnschlag, Ischämie, akuten und chronischen peripheren Gefäßerkrankungen besteht, ausgewählt sind.

4. Verwendung nach irgendeinem der Ansprüche 1 bis 3, wobei die native Oligonukleotidsequenz die folgende Oligonukleotidsequenz:
atgacttttg atgacctaaa gatccagact gtgaaggacc agcctgatga gaagtcaaat ggaaaaaaag ctaaaggtct tcagtttctt tactctccat ggtggtgcct ggctgctgcg actctagggg tcctttgcct gggattagta gtgaccatta tggtgctggg catgcaatta tcccaggtgt ctgacctcct aacacaagag caagcaaacc taactcacca gaaaaagaaa ctggagggac agatctcagc ccggcaacaa gcagaagaag cttcacagga gtcagaaaac gaactcaagg aaatgataga aacccttgct cggaagctga atgagaaatc caaagagcaa atggaacttc accaccagaa tctgaatctc caagaaacac tgaagagagt agcaaattgt tcagctcctt gtccgcaaga ctggatctgg catggagaaa actgttacct attttcctcg ggctcattta actgggaaaa cagccaagag aagtgcttgt ctttggatgc caagttgctg aaaattaata gcacagctga tctggacttc atccagcaag caatttccta ttccagtttt ccattctgga tggggctgtc tcggaggaac cccagctacc catggctctg ggaggacggt tctcctttga tgccccactt atttagagtc cgaggcgctg tctcccagac atacccttca ggtacctgtg catatataca acgaggagct gtttatgcgg aaaactgcat tttagctgcc ttcagtatat gtcagaagaa ggcaaaccta agagcacagt ga (SEQ ID NO:1) oder ihre komplementäre Sequenz umfasst.

5. Verwendung nach irgendeinem der Ansprüche 1-4, wobei die Oligonukleotidsequenz RNA oder DNA ist.

6. Verwendung nach Anspruch 5, wobei die Isoform markiert oder mit einer Oligonukleotidsequenz, die für einen Proteinmarker codiert, fusioniert ist.

7. Verwendung nach Anspruch 6, wobei das Protein aus der Gruppe, die aus Luciferase, grünes Fluoreszenzprotein (GFP) und dessen Varianten, fluoreszierenden Proteinen, c-myc oder Markierungsepitopen, die von monoklonalen oder polyklonalen Antikörpern erkannt werden, fluoreszierenden Farbstoffen, Biotin besteht, ausgewählt ist.

8. Verwendung nach irgendeinem der Ansprüche 1-7, wobei die Aminosäuresequenz die folgende Aminosäuresequenz umfasst:

9. Verwendung nach irgendeinem der Ansprüche 1-7, wobei die Oligonukleotidsequenz der alternativen Splice-Isoform für die Aminosäuresequenz wie in Anspruch 8 definiert codiert.

10. Verfahren zur Bestimmung des Risikos für kardiovaskuläre Erkrankungen, welches die folgenden Schritte umfasst:
a) quantitative Bestimmung der mRNA-Spiegel von LOX-1 und der alternativen Splice-Isoform, wie in irgendeinem der Ansprüche 2-9 definiert, in einer biologischen Probe;
b) Bestimmung des Werts des Verhältnisses R von nativem LOX-1-Transkript/Transkript der alternativen Splice-Isoform, **dadurch gekennzeichnet, dass** das Exon 5 von Nukleotid 565 bis Nukleotid 680 im Vergleich zu der entsprechenden nativen Oligonukleotidsequenz des Human-OLR1-Transkripts (NM 002543) ausgeschnitten ist, und des relativen Risikos auf der Basis der folgenden Werte:
| | |
|---|---|
| Niedriges Risiko | R < 0,6 |
| Mittleres Risiko | R zwischen 0,7-0,8 |
| Hohes Risiko | R > 0,9. |

11. Verfahren nach Anspruch 10, wobei der Nachweis von Schritt a) durch Echtzeit-PCR mittels mindestens einer Oligonukleotidsonde und mindestens einem Paar spezifischer Primer, welche spezifisch für mRNAs von nativem LOX-1 und/oder der alternativen Splice-Isoform wie in irgendeinem der Ansprüche 2-9 definiert sind, erfolgt.

12. Verfahren nach Anspruch 11, wobei der Nachweis erfolgt mittels zweier Oligonukleotidsonden und zwei Paaren von Primern, welche in der Lage sind, selektiv die beiden Isoformen, LOX-1 und die alternative Splice-LOXIN-Isoform, zu quantifizieren, wobei die Sonden die folgenden Oligonukleotidsequenzen umfassen:
i) Sonde LOX-1 5'-FAM-AGCTGATCTGGACTT-3' (SEQ ID NO: 3);
ii) Sonde LOXIN 5'-VIC-CAGCTGATCTGATTT-3' (SEQ ID NO: 4);
wobei die Primerpaare umfassen:
iii) Primer LOX-1 Fw 5'-TGCCAAGTTGCTGAAAATTAATAGC-3' (SEQ ID NO: 5);
iv) Primer LOX-1 Rv 5'-AACTGGAATAGGAAATTGCTTGCT-3' (SEQ ID NO: 6);
v) Primer LOXIN Fw 5'-TGCCAAGTTGCTGAAAATTAATAGC-3' (SEQ ID NO: 7;
vi) Primer LOXIN Rv 5'-TGAAGGGTATGTCTGGGAGACA-3' (SEQ ID NO: 8).

13. Verfahren nach irgendeinem der Ansprüche 10 bis 12, wobei die kardiovaskulären Erkrankungen aus der Gruppe, die aus Arteriosklerose, Myokard-Infarkt, arteriosklerotischer Koronarerkrankung, Gehirnschlag, Ischämie, akuten und chronischen peripheren Gefäßerkrankungen besteht, ausgewählt sind.

14. Verfahren nach irgendeinem der Ansprüche 10 bis 13, wobei die biologische Probe peripheres Blut ist.

15. Diagnostischer Kit, umfassend Oligonukleotidsonden und spezifische Primer für die native LOX-1-Isoform und für die alternative Splice-Isoform LOXIN, **dadurch gekennzeichnet, dass** das Exon 5 von Nukleotid 565 bis Nukleotid 680 im Vergleich zu der entsprechenden nativen Oligonukleotidsequenz des Human-OLRI-Transkripts (NM 002543) ausgeschnitten ist, zur Vorhersage des Risikos für eine kardiovaskuläre Erkrankung, wobei die Sonden die folgenden Oligonukleotidsequenzen umfassen:
i) Sonde LOX-1 5'-FAM-AGCTGATCTGGACTT-3' (SEQ ID NO: 3);
ii) Sonde LOXIN 5'-VIC-CAGCTGATCTGATTT-3' (SEQ ID NO: 4);
wobei die Primerpaare die folgenden Oligonukleotidsequenzen umfassen:
iii) Primer LOX-1 Fw 5'-TGCCAAGTTGCTGAAAATTAATAGC-3' (SEQ ID NO: 5);
iv) Primer LOX-1 Rv 5'-AACTGGAATAGGAAATTGCTTGCT-3' (SEQ ID NO: 6);
v) Primer LOXIN Fw 5'-TGCCAAGTTGCTGAAAATTAATAGC-3' (SEQ ID NO: 7;
vi) Primer LOXIN Rv 5'-TGAAGGGTATGTCTGGGAGACA-3' (SEQ ID NO: 8).

16. Kit nach Anspruch 15, wobei die kardiovaskulären Erkrankungen aus der Gruppe, die aus Arteriosklerose, Myokard-Infarkt, arteriosklerotischer Koronarkrankung, Gehirnschlag, Ischämie, akuten und chronischen peripheren Gefäßerkrankungen besteht, ausgewählt sind.

17. Verwendung einer Sonde für den in vitro-Nachweis der alternativen Splice-Isoform LOXIN, **dadurch gekennzeichnet, dass** das Exon 5 von Nukleotid 565 bis Nukleotid 680 im Vergleich zur entsprechenden nativen Oligonukleotidsequenz des Human-OLRI-Transkripts (NM 002543) ausgeschnitten ist, welche Sonde die folgende Nukleotidsequenz umfasst:
5'CAGCTGATCTGATTT-3' (SEQ ID NO: 4).

18. Verwendung nach Anspruch 17, wobei die Sonde mit einer Substanz markiert ist, welche aus der Gruppe, die aus einem Fluorophor, Radioisotop, einer lumineszierenden Substanz besteht, ausgewählt ist.

19. Pharmazeutische Zusammensetzung, umfassend die alternative Splice-Isoform wie in irgendeinem der Ansprüche 1-5 definiert oder den Vektor, welcher die Oligonukleotidsequenz der alternativen Splice-Isoform umfasst, oder die davon codierte Aminosäuresequenz als aktiven Wirkstoff zusammen mit einem oder mehreren pharmakologisch annehmbaren Adjuvanz/ien und/oder Exzipienten.

20. Pharmazeutische Zusammensetzung nach Anspruch 19, wobei die Aminosäuresequenz die folgende Aminosäuresequenz umfasst:

21. Pharmazeutische Zusammensetzung nach Anspruch 19, wobei die Oligonukleotidsequenz der alternativen Splice-Isoform für die Aminosäuresequenz wie in Anspruch 20 definiert codiert.

## Revendications

1. Utilisation d'une isoforme d'épissage alternatif du gène OLR1 codant pour le récepteur 1 des lipoprotéines de basse densité oxydées (LOX-1), **caractérisée en ce que** l'exon 5 à partir du nucléotide 565 au nucléotide 680 est excisé par rapport à la séquence oligonucléotidique native correspondante du transcrit de l'OLR1 humain (NM 002543) ou à sa séquence complémentaire, ou du vecteur comprenant la séquence oligonucléotidique de ladite isoforme d'épissage alternatif ou de la séquence d'acides aminés codée par ce moyen, pour la préparation d'un médicament anti-apoptotique inhibant l'instabilité des plaques d'athérosclérose.

2. Utilisation d'une isoforme d'épissage alternatif du gène OLR1 codant pour le récepteur 1 des lipoprotéines de basse densité oxydées (LOX-1), **caractérisée en ce que** l'exon 5 à partir du nucléotide 565 au nucléotide 680 est excisé par rapport à la séquence oligonucléotidique native correspondante du transcrit de l'OLR1 humain (NM 002543) ou à sa séquence complémentaire, ou du vecteur comprenant la séquence oligonucléotidique de ladite isoforme d'épissage alternatif ou de la séquence d'acides aminés codée par ce moyen, en tant que marqueur pour la détermination du risque de maladies cardiovasculaires, de préférence en déterminant le rapport entre la quantité de l'isoforme native correspondante et celle dudit marqueur.

3. Utilisation selon la revendication 2, dans laquelle lesdites maladies cardiovasculaires sont choisies dans le groupe constitué par l'athérosclérose, l'infarctus du myocarde, l'athérosclérose coronarienne, l'accident vasculaire cérébral, l'ischémie, les maladies vasculaires périphériques aiguës et chroniques.

4. Utilisation selon l'une quelconque des revendications 1 à 3, dans laquelle ladite séquence oligonucléotidique native comprend la séquence oligonucléotidique suivante : atgacttttg atgacctaaa gatccagact gtgaaggacc agcctgatga gaagtcaaat ggaaaaaaag ctaaaggtct tcagtttctt tactctccat ggtggtgcct ggctgctgcg actctagggg tcctttgcct gggattagta gtgaccatta tggtgctggg catgcaatta tcccaggtgt ctgacctcct aacacaagag caagcaaacc taactcacca gaaaaagaaa ctggagggac agatctcagc ccggcaacaa gcagaagaag cttcacagga gtcagaaaac gaactcaagg aaatgataga aacccttgct cggaagctga atgagaaatc caaagagcaa atggaacttc accaccagaa tctgaatctc caagaaacac tgaagagagt agcaaattgt tcagctcctt gtccgcaaga ctggatctgg catggagaaa actgttacct attttcctcg ggctcattta actgggaaaa cagccaagag aagtgcttgt ctttggatgc caagttgctg aaaattaata gcacagctga tctggacttc atccagcaag caatttccta ttccagtttt ccattctgga tggggctgtc tcggaggaac cccagctacc catggctctg ggaggacggt tctcctttga tgccccactt atttagagtc cgaggcgctg tctcccagac atacccttca ggtacctgtg catatataca acgaggagct gtttatgcgg aaaactgcat tttagctgcc ttcagtatat gtcagaagaa ggcaaaccta agagcacagt ga (SEQ ID NO:1), ou sa séquence complémentaire.

5. Utilisation selon l'une quelconque des revendications 1 à 4, dans laquelle la séquence oligonucléotidique est de l'ARN ou de l'ADN.

6. Utilisation selon la revendication 5, ladite isoforme étant marquée ou fusionnée avec une séquence oligonucléotidique codant pour un marqueur protéique.

7. Utilisation selon la revendication 6, dans laquelle ladite protéine est choisie dans le groupe constitué par la luciférase, la protéine fluorescente verte (GFP, *« Green fluorescent protein* ») et ses variantes, les protéines fluorescentes, les épitopes c-myc ou tag reconnus par les anticorps monoclonaux ou polyclonaux, les colorants fluorescents, la biotine.

8. Utilisation selon l'une quelconque des revendications 1 à 7, dans laquelle ladite séquence d'acides aminés comprend la séquence d'acides aminés suivante :

9. Utilisation selon l'une quelconque des revendications 1 à 7, dans laquelle la séquence oligonucléotidique de l'isoforme d'épissage alternatif code pour la séquence d'acides aminés telle que définie dans la revendication 8.

10. Procédé de détermination du risque de maladies cardiovasculaires comprenant les étapes suivantes :
a) la détection quantitative des taux d'ARNm de LOX-1 et de l'isoforme d'épissage alternatif telle que définie selon l'une quelconque des revendications 2 à 9 dans un échantillon biologique ;
b) la détermination de la valeur du rapport R transcrit de LOX-1 natif / transcrit de l'isoforme d'épissage alternatif **caractérisé en ce que** l'exon 5 à partir du nucléotide 565 au nucléotide 680 est excisé par rapport à la séquence oligonucléotidique native correspondante du transcrit de l'OLR1 humain (NM 002543) et du risque relatif sur la base des valeurs suivantes :
| | |
|---|---|
| Risque peu élevé | R <0,6 |
| Risque intermédiaire | R entre 0,7-0,8 |
| Risque élevé | R > 0,9. |

11. Procédé selon la revendication 10, dans lequel la détection de l'étape a) est réalisée au moyen de la PCR [Réaction en chaîne par polymérase] en temps réel par l'intermédiaire d'au moins une sonde oligonucléotidique et d'au moins une paire d'amorces spécifiques spécifique des ARNm du LOX-1 natif et/ou de l'isoforme d'épissage alternatif telle que définie selon l'une quelconque des revendications 2 à 9.

12. Procédé selon la revendication 11, dans lequel la détection est réalisée au moyen de deux sondes oligonucléotidiques et de deux paires d'amorces ayant la faculté de quantifier de façon sélective les deux isoformes LOX-1 et les isoformes d'épissage alternatif LOXIN, lesdites sondes comprenant les séquences oligonucléotidiques suivantes :
i) La sonde LOX-1 5'-FAM-AGCTGATCTGGACTT-3' (SEQ ID N° : 3) ;
ii) La sonde LOXIN 5'-VIC-CAGCTGATCTGATTT-3' (SEQ ID N° : 4) ;
lesdites paires d'amorces comprenant :
iii) L'amorce LOX-1 Fw 5'-TGCCAAGTTGCTGAAAATTAA TAGC-3' (SEQ ID N° : 5) ;
iv) L'amorce LOX-1 Rv 5'-AACTGGAATAGGAAATTGCT TGCT-3' (SEQ ID N° : 6) ;
v) L'amorce LOXIN Fw-5'-TGCCAAGTTGCTGAAAATTAA TAGC-3' (SEQ ID N° : 7) ;
vi) L'amorce LOXIN Rv 5'-TGAAGGGTATGTCTGGGA GACA-3' (SEQ ID N° : 8).

13. Procédé selon l'une quelconque des revendications 10 à 12, dans lequel lesdites maladies cardiovasculaires sont choisies dans le groupe constitué par l'athérosclérose, l'infarctus du myocarde, l'athérosclérose coronarienne, l'accident vasculaire cérébral, l'ischémie, les maladies vasculaires périphériques aiguës et chroniques.

14. Procédé selon l'une quelconque des revendications 10 à 13, dans lequel ledit échantillon biologique est du sang périphérique.

15. Trousse de diagnostic comprenant des sondes oligonucléotidiques et des amorces spécifiques de l'isoforme LOX-1 natif et de l'isoforme d'épissage alternatif LOXIN **caractérisée en ce que** l'exon 5 à partir du nucléotide 565 au nucléotide 680 est excisé par rapport à la séquence oligonucléotidique native correspondante du transcrit de l'OLR1 humain (NM 002543), pour la prédiction du risque de maladie cardiovasculaire, dans laquelle lesdites sondes comprennent les séquences oligonucléotidiques suivantes :
i) La sonde LOX-1 5'-FAM-AGCTGATCTGGACTT-3' (SEQ ID N° : 3) ;
ii) La sonde LOXIN 5'-VIC-CAGCTGATCTGATTT-3' (SEQ ID N° : 4) ;
lesdites paires d'amorces comprennent les séquences oligonucléotidiques suivantes :
iii) L'amorce LOX-1 Fw 5'-TGCCAAGTTGCTGAAAATTAA TAGC-3' (SEQ ID N° : 5) ;
iv) L'amorce LOX-1 Rv 5'-AACTGGAATAGGAAATTGCT TGCT-3' (SEQ ID N° : 6) ;
v) L'amorce LOXIN Fw 5'-TGCCAAGTTGCTGAAAATTAA TAGC-3' (SEQ ID N° : 7) ;
vi) L'amorce LOXIN Rv 5'-TGAAGGGTATGTCTGGGA GACA-3' (SEQ ID N° : 8).

16. Trousse selon la revendication 15, dans laquelle lesdites maladies cardiovasculaires sont choisies dans le groupe constitué par l'athérosclérose, l'infarctus du myocarde, l'athérosclérose coronarienne, l'accident vasculaire cérébral, l'ischémie, les maladies vasculaires périphériques aiguës et chroniques.

17. Utilisation d'une sonde pour la détection *in vitro* de l'isoforme d'épissage alternatif LOXIN **caractérisée en ce que** l'exon 5 à partir du nucléotide 565 au nucléotide 680 est excisé par rapport à la séquence oligonucléotidique native correspondante du transcrit de l'OLR1 humain (NM 002543), comprenant la séquence nucléotidique suivante :
5'- CAGGTCATTGATTT-3' (SEQ ID N° : 4).

18. Utilisation selon la revendication 17, dans laquelle ladite sonde est marquée au moyen d'une substance choisie dans le groupe constitué par fluorophore, radio-isotope, substance luminescente.

19. Composition pharmaceutique comprenant l'isoforme d'épissage alternatif telle que définie selon l'une quelconque des revendications 1 à 5, ou le vecteur comprenant la séquence oligonucléotidique de ladite isoforme d'épissage alternatif ou la séquence d'acides aminés codée par ce moyen, en tant que principe actif conjointement avec un ou plusieurs adjuvants et/ou excipients pharmacologiquement acceptables.

20. Composition pharmaceutique selon la revendication 19, dans laquelle ladite séquence d'acides aminés comprend la séquence d'acides aminés suivante :

21. Composition pharmaceutique selon la revendication 19, dans laquelle la séquence oligonucléotidique de l'isoforme d'épissage alternatif code pour la séquence d'acides aminés telle que définie dans la revendication 20.


### SEQUENCE LISTING '

<110> università degli studi di Tor Vergata
<120> Alternative splicing isoform of LOX-1 protein encoding gene, uses and method thereof
<130> PCT26410
<160> 17
<170> PatentIn version 3.2
<210> 1
   <211> 822
   <212> DNA
   <213> Artificial
<220>
   <223> Alternative splicing isoform of OLR-1 gene
<400> 1
   atgacttttg atgacctaaa gatccagact gtgaaggacc agcctgatga gaagtcaaat      60
   ggaaaaaaag ctaaaggtct tcagtttctt tactctccat ggtggtgcct ggctgctgcg     120
   actctagggg tcctttgcct gggattagta gtgaccatta tggtgctggg catgcaatta     180
   tcccaggtgt ctgacctcct aacacaagag caagcaaacc taactcacca gaaaaagaaa     240
   ctggagggac agatctcagc ccggcaacaa gcagaagaag cttcacagga gtcagaaaac     300
   gaactcaagg aaatgataga aacccttgct cggaagctga atgagaaatc caaagagcaa    360
   atggaacttc accaccagaa tctgaatctc caagaaacac tgaagagagt agcaaattgt     420
   tcagctcctt gtccgcaaga ctggatctgg catggagaaa actgttacct attttcctcg     480
   ggctcattta actgggaaaa cagccaagag aagtgcttgt ctttggatgc caagttgctg     540
   aaaattaata gcacagctga tctggacttc atccagcaag caatttccta ttccagtttt     600
   ccattctgga tggggctgtc tcggaggaac cccagctacc catggctctg ggaggacggt     660
   tctcctttga tgccccactt atttagagtc cgaggcgctg tctcccagac atacccttca     720
   ggtacctgtg catatataca acgaggagct gtttatgcgg aaaactgcat tttagctgcc     780
   ttcagtatat gtcagaagaa ggcaaaccta agagcacagt ga                        822
<210> 2
   <211> 189
   <212> PRT
   <213> Artificial
<220>
   <223> Alternative splicing isoform aminoacid sequence
<400> 2
   Met Thr Phe Asp Asp Leu Lys Ile Gln Thr Val Lys Asp Gln Pro Asp
   1               5                   10                  15
   Glu Lys Ser Asn Gly Lys Lys Ala Lys Gly Leu Gln Phe Leu Tyr Ser
               20                  25                  30
   Pro Trp Trp Cys Leu Ala Ala Ala Thr Leu Gly Val Leu Cys Leu Gly
           35                  40                  45
   Leu Val Val Thr Ile Met Val Leu Gly Met Gln Leu Ser Gln Val Ser
       50                  55                  60
   Asp Leu Leu Thr Gln Glu Gln Ala Asn Leu Thr His Gln Lys Lys Lys
   65                  70                  75                  80
   Leu Glu Gly Gln Ile Ser Ala Arg Gln Gln Ala Glu Glu Ala Ser Gln
                   85                  90                  95
   Glu Ser Glu Asn Glu Leu Lys Glu Met Ile Glu Thr Leu Ala Arg Lys
               100                 105                 110
   Leu Asn Glu Lys Ser Lys Glu Gln Met Glu Leu His His Gln Asn Leu
           115                 120                 125
   Asn Leu Gln Glu Thr Leu Lys Arg Val Ala Asn Cys Ser Ala Pro Cys
       130                 135                 140
   Pro Gln Asp Trp Ile Trp His Gly Glu Asn Cys Tyr Leu Phe Ser Ser
   145                 150                 155                 160
   Gly Ser Phe Asn Trp Glu Lys Ser Gln Glu Lys Cys Leu Ser Leu Asp
                   165                 170                 175
   Ala Lys Leu Leu Lys Ile Asn Ser Thr Ala Asp Leu Phe
               180                 185
<210> 3
   <211> 15
   <212> DNA
   <213> Artificial
<220>
   <223> LOX-1 probe
<400> 3
   agctgatctg gactt 15
<210> 4
   <211> 15
   <212> DNA
   <213> Artificial
<220>
   <223> Loxin probe
<400> 4
   cagctgatct gattt 15
<210> 5
   <211> 25
   <212> DNA
   <213> Artificial
<220>
   <223> Fw LOX-1 primer
<400> 5
   tgccaagttg ctgaaaatta atagc 25
<210> 6
   <211> 24
   <212> DNA
   <213> Artificial
<220>
   <223> Rv LOX-1 primer
<400> 6
   aactggaata ggaaattgct tgct 24
<210> 7
   <211> 25
   <212> DNA
   <213> Artificial
   .<220>
<223> Fw Loxin primer
<400> 7
   tgccaagttg ctgaaaatta atagc 25
<210> 8
   <211> 22
   <212> DNA
   <213> Artificial
<220>
   <223> Rv Loxin primer
<400> 8
   tgaagggtat gtctgggaga ca 22
<210> 9
   <211> 21
   <212> DNA
   <213> Artificial
<220>
   <223> OLR-1 Fw primer
<400> 9
   tgttgaagtt cgtgactgct t 21
<210> 10
   <211> 20
   <212> DNA
   <213> Artificial
<220>
   <223> OLR1-1 Rv primer
<400> 10
   ttctgcagcc agctaaatga 20
<210> 11
   <211> 33
   <212> DNA
   <213> Artificial
<220>
   <223> OLR1-xhoIF primer
<400> 11
   acagtcctcg aggtgagtgt tcatggatat ttg 33
<210> 12
   <211> 33
   <212> DNA
   <213> Artificial
<220>
   <223> OLR-1-EcoRIR primer
<400> 12
   tgtgtggata tcctgcaggt aggaaaaaca aaa 33
<210> 13
   <211> 21
   <212> DNA
   <213> Artificial
<220>
   <223> OLR1SEQF sequencing forward primer
<400> 13
   gtttcctatt ctttgctgaa c 21
<210> 14
   <211> 21
   <212> DNA
   <213> Artificial
<220>
   <223> OLR1SEQR sequencing reverse primer
<400> 14
   gtggggagta atgtttctga g 21
<210> 15
   <211> 30
   <212> DNA
   <213> Artificial
<220>
   <223> OLR-1 amplification primer
<400> 15
   ccgctcgaga tgacttttga tgacctaaag 30
<210> 16
   <211> 26
   <212> DNA
   <213> Artificial
<220>
   <223> OLR-1 amplification primer
<400> 16
   cgcggatcct gtgctcttag gtttgc 26
<210> 17
   <211> 29
   <212> DNA
   <213> Artificial
<220>
   <223> Loxin amplification primer
<400> 17
   cgcggatcca tcagatcagc tgtgctatt 29